# FASCICULE DE BREVET EUROPEEN

(11) **EP 2 882 360 B1**
(45) Date de publication et mention de la délivrance du brevet: **19.10.2022**
(21) Numéro de dépôt: 13762162.9
(22) Date de dépôt: 09.08.2013
(51) Int. Cl.: A61B 17/80

(54) **DISPOSITIF DE COAPTATION DE FRAGMENTS D'OS ET PROCEDES DE FABRICATION D'UN TEL DISPOSITIF**
VORRICHTUNG FÜR DIE KOAPTATION VON KNOCHENFRAGMENTEN UND VERFAHREN ZUR HERSTELLUNG EINER SOLCHEN VORRICHTUNG
DEVICE FOR COAPTATION OF BONE FRAGMENTS AND METHODS FOR PRODUCING SUCH A DEVICE

(30) Priorité: 13.08.2012 FR 1257787
(43) Date de publication de la demande: 17.06.2015
(73) Titulaire: TORNIER, 38330 Montbonnot-Saint-Martin (FR)
(72) Inventeur: IMPELLIZZERI, Frédéric, F-13300 Salon de Provence (FR)
(74) Mandataire: Lavoix
(86) Numéro de dépôt international: PCT/FR2013/051920
(87) Numéro de publication internationale: WO 2014/027160

(56) Documents cités:
- EP-A1- 0 171 884
- GB-A- 2 405 342
- US-A- 5 976 141
- US-A1- 2006 079 900
- US-A1- 2012 059 376
- US-A1- 2012 083 846

## Description

La présente invention concerne un dispositif de coaptation de fragments d'os. Elle concerne encore des procédés de fabrication d'un tel dispositif.

La coaptation des fragments d'os au moyen de plaques de titane ou autre matériau et de vis, pour réaliser une ostéosynthèse, est une intervention courante en chirurgie des os, par exemple en chirurgie orthopédique.

Pour l'obtention d'un bon résultat, un serrage permanent des plaques ou implants sur les fragments d'os assemblés par lesdits implants, est nécessaire. Il est donc indispensable que les vis ne puissent se dévisser et reculer, pour empêcher tout déplacement des implants par rapport aux fragments d'os.

D'autre part, il serait souvent souhaitable de pouvoir choisir l'orientation des vis par rapport aux plaques, aussi bien en fonction du positionnement et de la forme des fragments d'os à assembler, que pour améliorer la qualité de l'assemblage.

Enfin, il est primordial que la plaque d'ostéosynthèse ou d'ostéotomie se pose sur l'os avec des appuis ponctuels et non un appui surfacique, afin de sauvegarder le périoste. En effet, cette membrane vascularisée qui recouvre l'os sur toute sa surface (à l'exception du cartilage articulaire), contient les vaisseaux sanguins qui apportent les nutriments indispensables à la réparation de l'os. Les os dépourvus de périoste sont donc incapables de se régénérer et peuvent se nécroser, et c'est ce qui se produit avec un implant à appui surfacique.

Pour s'opposer au dévissage et au recul des vis, on a proposé (EP-0.345.133, FR-2.794.963) de loger des organes de blocage à l'entrée des trous de passage des vis prévus dans les plaques, afin de supprimer toute possibilité de mouvement axial de recul desdites vis, après leur vissage dans la matière osseuse. Par exemple, il est prévu, dans le document EP-0.345.133, d'utiliser des contre-vis filetées extérieurement et coopérant avec un filetage complémentaire ménagé à l'entrée des trous de passage des vis dont sont munies les plaques, de sorte que la tête desdites vis se trouve calée contre une contre-vis et que ces dernières ne peuvent se déplacer axialement par rapport auxdites plaques, ce blocage assurant ainsi la permanence de l'appui de la plaque sur les fragments d'os.

Ces dispositifs proposés par quelques fabricants, représentent, à ce jour, les solutions les plus sûres en termes de blocage. Toutefois, ces dispositifs relativement complexes nécessitent l'utilisation de plaques présentant une épaisseur relativement importante totalement incompatible avec une utilisation pour des interventions sur des os de la main ou des os du pied sans épaisseur de peau, pour lesquelles l'épaisseur des plaques doit être la plus réduite possible, compte tenu de la petite taille des os concernés et la faible épaisseur de la peau.

Dans le document EP-0.345.133, est encore montré un dispositif de solidarisation de deux éléments tels qu'un implant et un os, suivant lequel l'implant comporte des trous de passage des vis dont les axes sont orientés obliquement les uns par rapport aux autres, de sorte que les vis traversant ces trous ont des orientations rigoureusement imposées par la direction desdits axes. Un tel dispositif ne peut être envisagé que pour la réduction de fractures identiques, car autrement il faudrait prévoir autant de modèles de plaques que de cas possibles de fractures, ce qui est pratiquement impossible ; il n'offre en effet aucune possibilité de choisir l'orientation des vis en fonction des problèmes rencontrés en chirurgie orthopédique.

Dans le document WO-00/66012, est décrite une plaque pour ostéosynthèse pouvant être bloquée, suivant laquelle les vis et les trous de passage des vis prévus dans la plaque, sont munis, respectivement, d'un filetage de blocage et d'un profil d'engrènement censés permettre l'introduction des vis dans la plaque, de manière inclinée. La réalisation pratique d'un tel dispositif parait difficile et son efficacité n'a pas été établie, semble-t-il.

D'une façon générale, dans le domaine de l'ostéosynthèse de petits fragments d'os nécessitant l'utilisation de plaques de petites dimensions, les dispositifs actuellement présents sur le marché, ne rendent pas possible un débattement angulaire entre les vis et la plaque ni un verrouillage, de sorte que celles-ci se trouvent ainsi obligatoirement positionnées perpendiculairement à la plaque. Or, dans certains cas, il serait souhaitable de pouvoir incliner ou orienter une ou plusieurs vis, pour utiliser un ou des os de meilleure qualité pour le vissage, ou offrent de meilleures possibilités d'ancrage desdites vis.

Dans le document US-2011/0224737, est décrit un dispositif d'ostéosynthèse auto-bloquant comprenant une plaque munie de trous traversants réalisés en acier ou en titane, les pourtours de ces trous étant constitués par des inserts pourvus d'un passage axial et exécutés dans une matière biocompatible présentant des propriétés mécaniques autorisant un auto-taraudage de la paroi interne délimitant le passage axial desdits inserts au moyen de vis taraudeuses utilisables pour la fixation de ladite plaque sur des parties d'os ou des fragments d'os, lesdits inserts étant exécutés en polymère thermoplastique, par exemple en polyétheréthercétone (PEEK) et intégrés dans la plaque métallique par surmoulage ou par assemblage mécanique.

L'inconvénient de l'assemblage par un procédé de surmoulage est que l'adhérence entre les surfaces en contact de la plaque métallique et des inserts en matière plastique est minimale en raison de la non-compatibilité de ces matériaux, de sorte que l'accrochage chimique entre la plaque métallique et les inserts plastiques n'est pas parfait et que lesdits inserts peuvent se déplacer en n'assurant plus le bon positionnement des vis de verrouillage. Les procédés d'assemblage mécanique présentent le même inconvénient avec en plus la difficulté d'appliquer des méthodes d'assemblage mécanique automatisées, notamment lorsque la plaque n'est pas plane ou présente des formes complexes.

EP 0 171 884 A1, qui divulgue un dispositif selon le préambule de la revendication 1, enseigne principalement que des prothèses peuvent être fabriquées à partir de PEEK, de préférence renforcé par des fibres ou des particules dans une partie de cœur. Cette partie de cœur est recouverte par un revêtement de PEEK non-chargé, fixé à la partie de cœur par adhésion, par ancrage mécanique ou par une combinaison des deux. Pour éviter que la formation de trous expose les particules de renforcement, EP 0 171 884 A1 propose qu'une surface de la région de cœur, qui est exposée par usinage, soit fondue ou ramollie de manière que le polymère s'écoule sur les particules de renforcement éventuellement exposées. En variante, la surface exposée précitée peut être recouverte par un revêtement sur au moins les éléments de renforcement éventuellement exposés, par exemple par un procédé de moulage ultérieur. Il est également envisagé de prévoir une doublure sous forme d'inserts.

De son côté, GB 2 405 342 A divulgue une plaque de fixation osseuse renforcée de fibres, ayant une zone de fixation non renforcée, destinée à recevoir un élément de fixation osseuse, tel qu'une vis ou un clou. La plaque peut comprendre un corps principal dans lequel des inserts formant la zone de fixation non renforcée sont fixés, de préférence par soudage par ultrasons. En variante, elle peut comprendre une structure stratifiée avec une ou plusieurs couches de matériau renforcé de fibres, superposées à des couches de matériau non renforcé, formant la zone de fixation non renforcée.

L'invention a notamment pour but de remédier aux insuffisances susmentionnées des dispositifs d'ostéosynthèse utilisant des plaques et des vis, notamment en raison du fait que les dispositifs existant pour de l'orthopédie lourde (traitement des gros traumatismes), ne sont pas transposables à la chirurgie des mains et des pieds pour laquelle la dimension des plaques utilisables se réduit considérablement.

Selon l'invention, ce but est atteint grâce à un dispositif de coaptation de parties d'os ou de fragments d'os, tel que défini à la revendication 1.

Il revient sensiblement au même de dire que l'objet de l'invention est une plaque de coaptation destinée à être fixée sur des fragments osseux ou que c'est un dispositif de coaptation de parties d'os ou de fragments d'os, ou encore qu'il s'agit d'une plaque d'ostéosynthèse.

Ainsi l'invention met en oeuvre deux matériaux polymères présentant une compatibilité telle que, par moulage (moulage simultané ou surmoulage) il se produise une interdiffusion ou interfusion telle qu'il y ait une continuité rhéologique entre la partie de support d'une part, et les zones ou inserts d'autre part. Il faut bien comprendre qu'ainsi, les inserts précités ne sont plus clairement identifiables comme dans les solutions connues. Il en résulte une tenue mécanique de très bonne qualité empêchant tout mouvement relatif entre la partie de support et les zones et inserts, même en ayant initialement donné à ces zones et inserts une géométrie simple (elles peuvent ainsi être simplement cylindriques). Pourtant le rôle de partie de support est efficacement assuré par le fait que le matériau polymère qui la constitue est un matériau plus rigide que le matériau des zones ou inserts.

La compatibilité précitée entre les matériaux constitutifs de la partie de support d'une part, et des zones ou inserts d'autre part, peut se définir en termes de rhéologie, et/ou de retrait, et/ou d'adhésion et/ou de propriétés thermiques ; en fait ces diverses notions expriment de manière différente la capacité à s'interpénétrer par moulage.

Une autre manière de caractériser la différence entre la partie de support et les zones ou inserts est de dire que la partie de support est plus dure que les zones ou inserts.

Selon un mode d'exécution, le premier polymère dans lequel est exécutée la partie de support de la plaque de coaptation, (donc en dehors des zones ou inserts constitués par le second polymère de moindre rigidité), est chargé en fibres de Carbone implantables.

De manière avantageuse, ce premier polymère peut comporter une charge de durcissement (par exemples les fibres de carbone précitées) dans une matrice formée du second polymère ; cela favorise ladite continuité rhéologique précitée.

Ainsi, selon une disposition avantageuse de l'invention, les zones ou inserts de moindre dureté sont exécutés en Polyétheréthercétone (PEEK) et le polymère dans lequel est exécutée la partie de support de la plaque de coaptation, en dehors des zones ou inserts de moindre dureté, est exécutée en Polyétheréthercétone (PEEK) chargé en fibres de Carbone implantables.

L'invention a notamment pour avantages :
- de procurer une adhésion maximale assurant ainsi une cohésion optimale de la liaison entre les zones ou inserts de moindre dureté et la partie de support de la plaque ;
- de donner toute latitude au concepteur et au producteur de ces dispositifs quant à la géométrie des parties de support, qui peuvent donc avoir une forme approchant au mieux la zone où une coaptation est souhaitée (un résultat peut être un confort appréciable pour le patient par rapport aux solutions connues ou les inserts sont portés par une plaque métallique, qui n'est généralement disponible que dans des formes simples, planes ou courbées, mais ne présentant généralement pas plusieurs courbures dans des plans différents - notamment à propos des surfaces dites « gauches ») ;
- de supprimer ou de minimaliser les opérations manuelles ou mécaniques de positionnement des inserts dans une plaque d'ostéosynthèse ou d'ostéotomie, en permettant ainsi un gain de productivité.

Grâce aux dispositions caractéristiques ci-dessus, la tête auto-taraudeuse des vis réalise son propre sillon hélicoïdal récepteur dans le pourtour des trous dans lesquels elles sont engagées, de sorte que lesdites vis se trouvent ensuite automatiquement bloquées dans la plaque lorsque leur tête se trouve serrée dans son logement.

D'autre part, la plaque d'ostéosynthèse selon l'invention autorise une angulation sélective des vis par rapport à l'axe des trous de ladite plaque, en fonction des besoins.

Selon une autre disposition intéressante, la plaque présente, en sous-face (ou face destinée à être placée en regard des fragments osseux dont on veut réaliser la coaptation), au niveau des zones ou inserts de moindre dureté, des surépaisseurs locales.

Pour la fabrication d'un dispositif de coaptation du type précité, l'invention propose un procédé tel que défini à la revendication 7.

Pour ce faire il suffit de choisir des matériaux polymères biocompatibles capables de fondre ensemble, et de s'interpénétrer au moment de leurs refroidissements.

De manière avantageuse, les deux matériaux polymère diffèrent seulement par le fait que l'un des matériaux polymères contient une charge de durcissement, par exemple formée par des fibres de carbone.

Selon un premier exemple de réalisation, le dispositif de coaptation selon l'invention est réalisé par un procédé de surmoulage (on réalise l'insert en un premier temps et on moule le matériau plus rigide en un second temps).

Selon un autre exemple d'exécution, le dispositif de coaptation selon l'invention est réalisé par un procédé d'injection bi-matière (cela revient à dire qu'on réalise simultanément l'insert (ou les inserts lorsqu'il y en plusieurs) et la partie de support).

Ainsi les dispositifs de coaptation de l'invention peuvent être obtenus par surmoulage, ou par injection bi-matière.

Sont connues depuis plusieurs années des techniques permettant d'obtenir des pièces avec inserts.

Le surmoulage consiste à mettre en place, dans l'empreinte d'une cavité d'un moule d'injection, une pièce A (ou insert) et à injecter une matière B. Le remplissage de l'empreinte permet de réaliser le surmoulage de la pièce A avec la matière B (définition tirée des « Techniques de l'ingénieur ^{©} »).

L'injection multi-matière permet d'injecter successivement ou simultanément plusieurs polymères pour réaliser une pièce complexe. Le premier module injecté constitue l'insert, les suivants les surmoulages (définition tirée des « Techniques de l'ingénieur ^{©} »).

Toutefois, les notions de surmoulage ou d'injection multi-matière n'impliquent pas en soi qu'il y ait une continuité rhéologique entre les deux matériaux, contrairement à ce que prévoit l'invention.

Selon l'invention, les matériaux sont choisis en sorte que le matériau constitutif de la partie de support ait une température de fusion supérieure à celle du matériau constitutif des inserts, de manière à ce que, lors du moulage de la partie de support, il y ait une fusion (en pratique uniquement superficielle) des inserts de manière à permettre l'obtention de la continuité rhéologique (le temps d'injection et de refroidissement est choisi en sorte que les inserts ne soient pas maintenus trop longtemps à la température de fusion de l'autre matériau).

Des cas particulièrement intéressants consistent à utiliser le matériau le plus malléable comme matrice du matériau le plus rigide, cette matrice contenant une charge de durcissement, telle que du carbone, de préférence sous forme de fibres.

Selon un premier exemple de mise en œuvre, un dispositif de coaptation du type précité est obtenu par un procédé de surmoulage suivant lequel :
- on place dans un moule, au moins une pièce préformée ou insert exécuté dans un polymère biocompatible et malléable ;
- on injecte, dans ce moule, autour de ladite pièce préformée, un matériau formé du même polymère biocompatible mais chargé en Carbone, et présentant, après polymérisation/moulage, une rigidité supérieure à celle de ladite pièce préformée ou insert ;
- on laisse refroidir la pièce avant de l'éjecter.

Selon une caractéristique avantageuse, le matériau biocompatible et malléable constituant l'insert est du Polyétheréthercétone (PEEK naturel).

Selon une autre caractéristique avantageuse, le matériau biocompatible injecté autour de l'insert pour constituer la plaque de support est du PEEK chargé en fibres de Carbone implantables.

Selon une caractéristique intéressante du procédé de l'invention, l'injection se fait dans un moule dont la température est comprise entre 140°C et 220°C.

Selon un exemple de mise en œuvre, la température du moule est de 175°C.

Selon une autre caractéristique intéressante du procédé de l'invention, le polymère biocompatible chargé en fibres de Carbone est injecté à une température comprise entre 350°C et 440°C.

Selon un exemple de réalisation, le polymère biocompatible chargé en fibres de Carbone est injecté à une température de 395°C.

Selon une autre caractéristique intéressante du procédé de l'invention, le polymère biocompatible chargé en fibres de Carbone est injecté à une vitesse comprise entre 50 g/sec et 750 g/sec.

Selon un exemple de réalisation, le polymère biocompatible chargé en fibres de Carbone est injecté à une vitesse de 300 g/sec.

Selon une autre caractéristique intéressante du procédé de l'invention, le polymère biocompatible chargé en fibres de Carbone est injecté à une pression comprise entre 500 et 2000 Bars.

Selon un exemple de réalisation, le polymère biocompatible chargé en fibres de Carbone est injecté à une pression de 1000 Bars.

Selon encore une caractéristique intéressante du procédé, le dispositif obtenu nécessite un temps de refroidissement compris entre 10 secondes et 30 secondes.

Selon un exemple de mise en œuvre, le temps de refroidissement du dispositif obtenu est de 20 secondes.

Selon un deuxième exemple de mise en œuvre, le dispositif de coaptation selon l'invention est réalisé par un procédé d'injection bi-matière suivant lequel :
- on injecte dans un moule, muni de deux points d'injection pour l'alimentation de chacune des matières, un polymère biocompatible et malléable pour fabriquer lesdits inserts et un matériau polymère formé du même polymère biocompatible mais chargé en fibre de Carbone pour fabriquer la partie de support, présentant après polymérisation/moulage une rigidité supérieure à celle de l'insert ;
- on laisse refroidir la pièce, i.e. le dispositif, avant de l'éjecter.

Selon une caractéristique avantageuse, le matériau biocompatible et malléable constituant l'insert est du Polyétheréthercétone (PEEK naturel).

Selon une autre caractéristique avantageuse, le matériau biocompatible injecté autour de l'insert pour constituer la plaque de support est du PEEK chargé en fibres de Carbone implantables.

Selon une caractéristique intéressante du procédé de l'invention, l'injection se fait dans un moule dont la température est comprise entre 140°C et 220°C.

Selon un exemple de mise en œuvre, la température du moule est de 175°C.

Selon une autre caractéristique intéressante du procédé de l'invention, le polymère biocompatible chargé en fibres de Carbone est injecté à une température comprise entre 350°C et 440°C.

Selon un exemple de réalisation, le polymère biocompatible chargé en fibres de Carbone est injecté à une température de 395°C.

Selon une autre caractéristique intéressante du procédé de l'invention, le polymère biocompatible chargé en fibres de Carbone est injecté à une vitesse comprise entre 50 g/sec et 750 g/sec.

Selon un exemple de réalisation, le polymère biocompatible chargé en fibres de Carbone est injecté à une vitesse de 300 g/sec.

Selon une autre caractéristique intéressante du procédé de l'invention, le polymère biocompatible chargé en fibres de Carbone est injecté à une pression comprise entre 500 et 2000 Bars.

Selon un exemple de réalisation, le polymère biocompatible chargé en fibres de Carbone est injecté à une pression de 1000 Bars.

Selon encore une caractéristique intéressante du procédé, le dispositif obtenu nécessite un temps de refroidissement compris entre 10 secondes et 30 secondes.

Selon un exemple de mise en œuvre, le temps de refroidissement du dispositif obtenu est de 20 secondes.

Le dispositif et les procédés selon l'invention procurent plusieurs avantages intéressants. Notamment :
- de proposer une fabrication plus simple, plus rapide et moins couteuse de dispositifs complexes constitués des deux matériaux en question ;
- de permettre de rétablir la rigidité du squelette grâce à la plaque exécutée en PEEK chargé en fibres de Carbone pouvant supporter des charges importantes tout en permettant le verrouillage des têtes de vis par la compression du matériau malléable dans le filet desdites têtes de vis grâce aux inserts en PEEK naturel ;
- d'assurer le verrouillage des vis de fixation des plaques d'ostéosynthèse même de petites tailles, avec des angles de +/- 10° et une compression optimale ;
- d'induire des surépaisseurs locales de la plaque au niveau des inserts en PEEK permettant, lorsqu'elle est placée sur un os, de préserver le périoste de l'os nécessaire à une bonne reconstruction de l'os.

De plus, le dispositif de coaptation selon l'invention entièrement exécutée en PEEK procure de nombreux avantages par rapport aux plaques métalliques à savoir :
- d'éviter les accroches osseuses qui apparaissent avec des plaques en métal et qui posent problème au moment du retrait de la plaque de coaptation ;
- la possibilité d'avoir des inserts injectés par surmoulage ce qui est difficilement réalisable sur une plaque en métal, l'étape de pliage ayant une mauvaise répétabilité. En effet, des résultats acceptables pourraient être obtenus avec le procédé de surmoulage en utilisant des cotes très précises de sorte à éviter les bavures occasionnées par le fluage de la matière, ce qui rendrait le procédé de fabrication trop complexe et trop couteux.

Le dispositif de coaptation selon l'invention et ses procédés de fabrications répondent donc parfaitement aux attentes, notamment des chirurgiens, en termes de facilité de fabrication, de facilité de mise en place et de fiabilité à l'usage.

Les buts, caractéristiques et avantages ci-dessus, et d'autres encore, ressortiront mieux de la description détaillée qui suit et des dessins annexés dans lesquels :

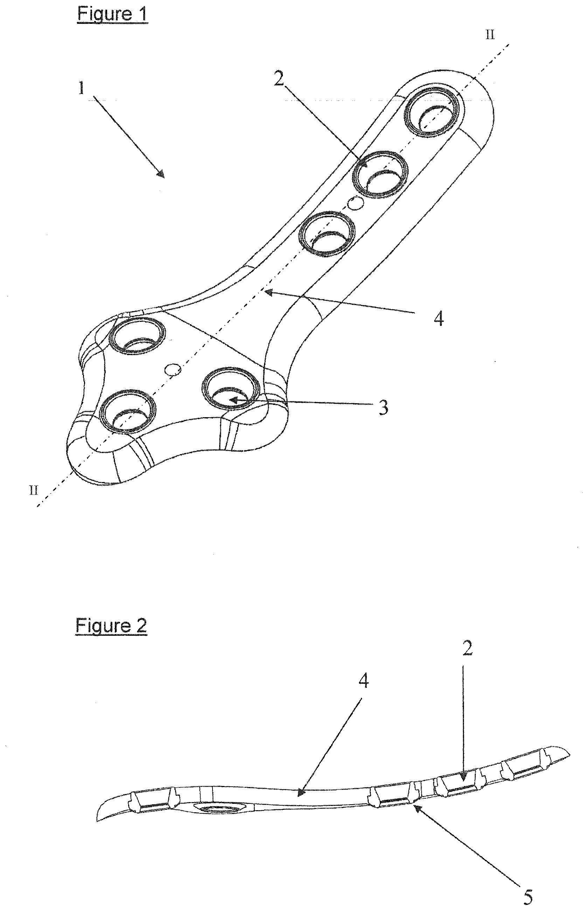

La figure 1 est une vue en perspective d'un premier exemple de réalisation d'une plaque d'ostéosynthèse selon l'invention, pour une utilisation au niveau de la malléole médiale.
La figure 2 est une vue en coupe selon la ligne II-II de la figure 1.
La figure 3 est une vue en perspective d'un autre exemple plus complexe de plaque d'ostéosynthèse selon l'invention, pour une utilisation au niveau de l'articulation talo-naviculaire.
La figure 4 est une vue de détail du dispositif d'ostéosynthèse selon l'invention.

On se réfère auxdits dessins pour décrire un exemple intéressant quoique nullement limitatif, de réalisation de la plaque de coaptation selon l'invention et de mise en œuvre des procédés de surmoulage et d'injection bi-matière.

Dans le présent exposé et dans les revendications, l'expression « sous-face » désigne la face destinée à être placée en regard des fragments osseux dont on veut réaliser la coaptation.

Dans les dessins, des interfaces sont visualisés entre les zones ou inserts d'une part, et leur partie de support ; il faut bien comprendre que ces interfaces ne sont visualisées que pour permettre la compréhension de ces dessins, alors que, puisqu'il y a une fusion moléculaire partielle d'un matériau à l'autre, ces interfaces ne sont en fait pas nettement matérialisées.

### Dispositif de coaptation

Le dispositif de coaptation selon l'invention présente une forme appropriée à l'usage auquel il est destiné. Il est prévu pour être fixé sur des fragments osseux, au moyen de vis (non représentées), pour assurer leur coaptation. Il comprend une plaque 1 comportant une partie, dite partie de support 4, est munie d'au moins une zone ou insert 2 pourvu d'un trou 3 exécuté dans un polymère biocompatible malléable présentant des propriétés mécaniques autorisant un auto-taraudage du pourtour desdits trous au moyen de vis filetées utilisables pour la fixation dudit dispositif, la partie restante ou partie de support 4 de ladite plaque de coaptation 1 de fragments d'os étant exécutée dans un polymère biocompatible chargé en Carbone et présentant, après polymérisation/moulage, une rigidité supérieure à celle de la ou des zones ou inserts 2.

Avantageusement, la plaque comporte plusieurs inserts 2.

Selon une caractéristique intéressante, les zones ou inserts 2 sont exécutés en Polyétheréthercétone naturel et la partie de support 4 est réalisée en PEEK chargé en fibres de Carbone implantables.

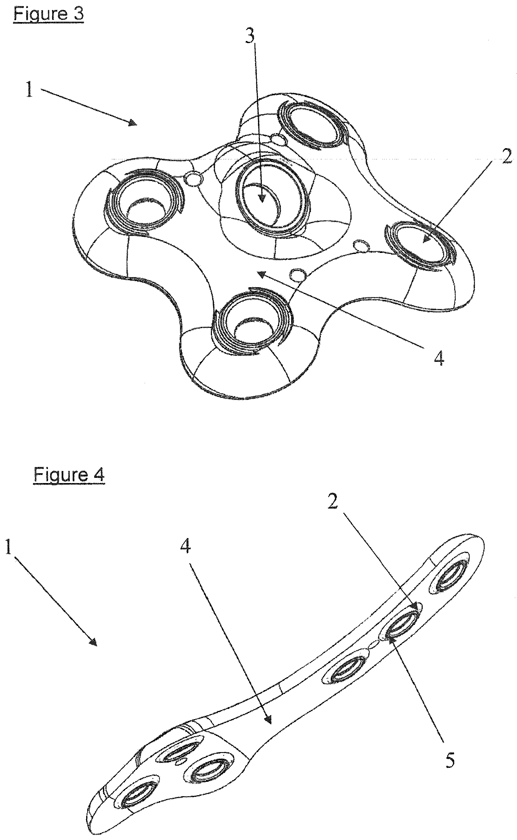

De manière avantageuse et comme illustré à la figure 4, la plaque de coaptation 1 présente, en sous-face, au niveau des inserts 2, des surépaisseurs locales 5. En effet, les inserts 2 permettent d'induire ces surépaisseurs locales 5 de sorte que ladite plaque est placée sur les fragments d'os dont on veut réaliser la coaptation, le périoste desdits fragments osseux est préservé, ce qui permet une meilleure reconstruction osseuse.

Selon un premier exemple de réalisation, le dispositif de coaptation 1 selon l'invention est réalisé par un procédé de surmoulage décrit ci-après.

Selon un autre exemple d'exécution, le dispositif de coaptation selon l'invention est réalisé par un procédé d'injection bi-matière décrit ci-après.

### Le procédé de surmoulage

Un tel dispositif de coaptation comprenant une ou plusieurs des caractéristiques susmentionnées, est obtenu par un procédé de surmoulage suivant lequel :
- on place dans un moule, au moins une pièce préformée ou insert exécuté dans un polymère biocompatible et malléable ;
- on injecte dans ce moule, autour de ladite pièce préformée, un polymère biocompatible chargé en fibres de Carbone, et présentant, après polymérisation/moulage, une rigidité supérieure à celle de ladite pièce préformée ou insert ;
- on laisse refroidir la pièce avant de l'éjecter.

Selon une caractéristique avantageuse, le matériau biocompatible et malléable constituant l'insert est du Polyétheréthercétone (PEEK naturel).

Selon une autre caractéristique avantageuse, le matériau biocompatible injecté autour de l'insert pour constituer la partie de support 4 de la plaque 1 est du PEEK chargé en fibres de Carbone implantables.

L'insert en PEEK naturel et le PEEK chargé en fibres de Carbone constituant la partie restante de la plaque étant compatibles, il y donc liaison chimique entre les deux matériaux et une fusion partielle optimisant la solidité de cette liaison.

La solidité de cette liaison pouvant être affectée par différents facteurs, comme la température à l'interface, la propreté de l'insert ou la température de fusion, ou la géométrie de l'interface, il est nécessaire de respecter certaines conditions.

Ainsi, selon le procédé de l'invention, l'injection se fait dans un moule dont la température est comprise entre 140°C et 220°C, et plus particulièrement à une température de 175°C.

Selon une autre caractéristique du procédé de l'invention, le polymère biocompatible chargé en Carbone est injecté à une température comprise entre 350°C et 440°C. Avantageusement il est injecté à une température de 395°C.

Le polymère biocompatible chargé en Carbone est injecté à une vitesse comprise entre 50 g/sec et 750 g/sec. Par exemple, il est injecté à une vitesse de 300 g/sec.

Selon le procédé de l'invention, il est préférable d'éviter les pressions de maintien extrêmement élevées. Ainsi, le polymère biocompatible chargé en Carbone est injecté à une pression comprise entre 500 et 2000 Bars Avantageusement, il est injecté à une pression de 1000 Bars.

Afin d'éviter un retrait excessif (c'est-à-dire la rétractation de la matière lors de son refroidissement en fin de surmoulage) lors de la phase finale de production et donc d'éviter une possibilité de perte fonctionnelle, le dispositif obtenu nécessite un temps de refroidissement compris entre 10 secondes et 30 secondes. De préférence, le temps de refroidissement du dispositif obtenu est de 20 secondes.

### Le procédé de bi-iniection

Selon un autre exemple de mise en œuvre, un dispositif de coaptation selon l'invention est obtenu par un procédé d'injection bi-matière suivant lequel :
- on injecte dans un moule, muni de deux points d'injection pour l'alimentation de chacune des matières, un polymère biocompatible (PEEK) pour fabriquer lesdits inserts et un polymère biocompatible chargé en fibre de Carbone pour fabriquer la partie de support 4 de la plaque 1, présentant après polymérisation/moulage une rigidité supérieure à celle de l'insert ;
- on laisse refroidir la pièce avant de l'éjecter.

Selon une caractéristique avantageuse, le matériau biocompatible et malléable constituant l'insert est du Polyétheréthercétone (PEEK).

Selon une autre caractéristique avantageuse, le matériau biocompatible injecté autour de l'insert pour constituer la partie de support 4 de la plaque 1 est du PEEK chargé en fibres de Carbone implantables.

L'insert en PEEK et le PEEK chargé en Carbone constituant la partie restante de la plaque, i.e. la partie de support, étant compatibles, il y donc liaison chimique entre les deux matériaux et une fusion partielle optimisant la solidité de cette liaison.

La solidité de cette liaison pouvant être affectée par différents facteurs, comme la température à l'interface, la propreté de l'insert ou la température de fusion, il est nécessaire de respecter certaines conditions.

Ainsi, selon le procédé de l'invention, l'injection se fait dans un moule dont la température est comprise entre 140°C et 220°C, et plus particulièrement à une température de 175°C.

Selon une autre caractéristique du procédé de l'invention, le polymère biocompatible chargé en Carbone est injecté à une température comprise entre 350°C et 440°C, Avantageusement il est injecté à une température de 395°C.

Le polymère biocompatible chargé en Carbone est injecté à une vitesse comprise entre 50 g/sec et 750 g/sec. Par exemple, il est injecté à une vitesse de 300 g/sec.

Selon le procédé de l'invention, il est impératif d'éviter les pressions de maintien extrêmement élevées. Ainsi, le polymère biocompatible chargé en Carbone est injecté à une pression comprise entre 500 et 2000 Bars Avantageusement, il est injecté à une pression de 1000 Bars.

Afin d'éviter un retrait excessif (c'est-à-dire la rétractation de la matière lors de son refroidissement en fin d'injection) lors de la phase finale de production et donc d'éviter une possibilité de perte fonctionnelle, le dispositif obtenu nécessite un temps de refroidissement compris entre 10 secondes et 30 secondes. De préférence, le temps de refroidissement du dispositif obtenu est de 20 secondes.

## Revendications

1. Dispositif de coaptation de parties d'os ou de fragments d'os, comprenant une plaque (1) monobloc venue de moulage dont une partie de support (4) est réalisée dans un premier polymère biocompatible et comportant au moins une zone ou insert (2) muni d'un trou traversant (3), ladite au moins une zone ou insert étant réalisé dans un deuxième polymère biocompatible qui est plus malléable que le premier polymère, ladite au moins une zone ou insert présentant des propriétés mécaniques autorisant un auto-taraudage de la surface interne des trous, au moyen de vis utilisables pour la fixation de ladite plaque sur des tissus osseux, ce dispositif étant **caractérisé en ce que** le premier polymère a une température de fusion supérieure à celle du deuxième polymère, et **en ce que** la partie de support (4) et ladite au moins une zone ou insert (2) présentent une continuité rhéologique entre eux résultant d'une fusion moléculaire partielle entre eux, qui est obtenue par une fusion uniquement superficielle de ladite au moins une zone ou insert lors du moulage de la partie de support.

2. Dispositif de coaptation de parties d'os ou de fragments d'os selon la revendication 1, **caractérisé en ce que** le premier polymère dans lequel est exécutée la partie de support (4), en dehors de ladite au moins une zone ou insert (2) constitué par le second polymère, est chargé en fibres de Carbone implantables.

3. Dispositif de coaptation de parties d'os ou de fragments d'os selon la revendication 1 ou la revendication 2, **caractérisé en ce que** le premier polymère comporte une charge de durcissement dans une matrice formée du deuxième polymère.

4. Dispositif de coaptation de parties d'os ou de fragments d'os suivant l'une des revendications 1 à 3, **caractérisé en ce que** ladite au moins une zone ou insert (2) est exécuté en Polyétheréthercétone (PEEK), et le premier polymère dans lequel est exécutée la partie de support (4), en dehors de ladite au moins une zone ou insert, est exécuté en Polyétheréthercétone (PEEK) chargé en fibres de Carbone implantables.

5. Dispositif de coaptation de parties d'os ou de fragments d'os selon l'une quelconque des revendications 1 à 4, **caractérisé en ce que** la plaque (1) présente, en sous-face, au niveau de ladite au moins une zone ou insert (2), des surépaisseurs locales (5).

6. Dispositif de coaptation de parties d'os ou de fragments d'os selon l'une des revendications 1 à 5, **caractérisé en ce que** plusieurs zones ou inserts (2) sont prévus.

7. Procédé de fabrication d'un dispositif de coaptation selon l'une quelconque des revendications 1 à 6, selon lequel on forme dans un moule au moins un insert (2) en un matériau polymère qui est biocompatible et malléable et on injecte un autre matériau polymère qui est biocompatible, pour former une partie de support (4) d'une plaque (1) du dispositif de coaptation, **caractérisé en ce que** ledit autre matériau polymère a une température de fusion supérieure à celle du matériau polymère biocompatible malléable et est injecté autour de ce matériau polymère biocompatible malléable après ou en même temps que ledit au moins un insert (2) est formé, dans des conditions de pression, de durée et de température telles qu'il se produise une continuité rhéologique entre les deux matériaux polymères résultant en une fusion moléculaire partielle entre les deux matériaux polymères, obtenue par une fusion uniquement superficielle dudit au moins un insert lors du moulage de la partie de support, en sorte d'obtenir monobloc la plaque (1).

8. Procédé selon la revendication 7, selon lequel les deux matériaux polymères diffèrent seulement par le fait que ledit autre matériau polymère contient une charge de durcissement.

9. Procédé selon la revendication 7 ou la revendication 8, selon lequel le procédé est opéré par surmoulage, et selon lequel :
- on place dans un moule, au moins une pièce préformée ou insert (2) exécuté dans le matériau polymère biocompatible et malléable ;
- on injecte dans ce moule, autour de ladite pièce préformée ou insert (2), un matériau formé du même polymère biocompatible mais chargé en fibres de Carbone, et présentant, après polymérisation/moulage, une rigidité supérieure à celle de ladite pièce préformée ou insert ; et
- on laisse refroidir le dispositif avant de l'éjecter.

10. Procédé selon la revendication 7 ou la revendication 8, selon lequel le procédé est opéré par injection bi-matière, et selon lequel :
- on injecte dans un moule, muni de deux points d'injection pour l'alimentation de chacune des deux matériaux polymères, le matériau polymère biocompatible et malléable pour fabriquer ledit au moins un insert (2) et un matériau polymère formé du même polymère biocompatible mais chargé en fibre de Carbone pour fabriquer la partie de support (4), présentant après polymérisation/moulage une rigidité supérieure à celle dudit au moins un insert (2) ; et
- on laisse refroidir le dispositif avant de l'éjecter.

11. Procédé selon l'une quelconque des revendications 9 à 10, selon lequel le matériau polymère biocompatible et malléable constituant ledit au moins un insert (2) est du Polyétheréthercétone (PEEK naturel), et selon lequel le matériau biocompatible injecté autour dudit au moins un insert (2) pour constituer la partie de support (4) est du PEEK chargé en fibres de Carbone implantables.

12. Procédé selon l'une quelconque des revendications 9 à 11, selon lequel l'injection se fait dans un moule dont la température est comprise entre 140°C et 220°C, et plus particulièrement à une température de 175°C.

13. Procédé selon l'une quelconque des revendications 9 à 12, selon lequel le matériau polymère biocompatible chargé en fibres de Carbone est injecté à une température comprise entre 350°C et 440°C, et plus particulièrement à une température de 395°C.

14. Procédé selon l'une quelconque des revendications 9 à 13, selon lequel le matériau polymère biocompatible chargé en fibres de Carbone est injecté à une vitesse comprise entre 50 g/sec et 750 g/sec, et plus particulièrement à une vitesse de 300 g/sec.

15. Procédé selon l'une quelconque des revendications 9 à 14, selon lequel le matériau polymère biocompatible chargé en fibres de Carbone est injecté à une pression comprise entre 500 et 2000 Bars, et plus particulièrement à une pression de 1000 Bars.

16. Procédé selon l'une quelconque des revendications 9 à 15, selon lequel le dispositif obtenu nécessite un temps de refroidissement compris entre 10 secondes et 30 secondes, et plus particulièrement le temps de refroidissement du dispositif obtenu est de 20 secondes.

## Patentansprüche

1. Vorrichtung für die Koadaptation von Knochenteilen oder Knochenfragmenten, umfassend eine geformte einteilige Platte (1), von der ein tragender Teil (4) aus einem ersten biologisch kompatiblen Polymer hergestellt ist und mindestens eine oder Zone oder einen Einsatz (2) aufweist, der mit einem Durchgangsloch (3) ausgestattet sind, wobei die mindestens eine Zone oder der Einsatz aus einem zweiten biologisch kompatiblen Polymer hergestellt ist, das formbarer als das erste Polymer ist, wobei die mindestens eine Zone oder der Einsatz mechanische Eigenschaften aufweist, die ein Selbstschneiden der inneren Oberfläche der Löcher mittels Schrauben gestatten, die für die Befestigung der Platte auf Knochengeweben verwendbar sind, wobei diese Vorrichtung **dadurch gekennzeichnet ist, dass** das erste Polymer eine Schmelztemperatur über der des zweiten Polymers hat und dass der tragende Teil (4) und die mindestens eine Zone oder der Einsatz (2) eine rheologische Kontinuität untereinander aufweisen, die aus einer teilweisen molekularen Verschmelzung zwischen ihnen resultiert, die durch eine nur oberflächliche Verschmelzung der mindestens einen Zone oder des Einsatzes beim Formen des tragenden Teils erhalten wird.

2. Vorrichtung für die Koadaptation von Knochenteilen oder Knochenfragmenten nach Anspruch 1, **dadurch gekennzeichnet, dass** dem ersten Polymer, aus dem der tragende Teil (4) außerhalb der mindestens eine Zone oder des Einsatzes (2), der aus dem zweiten Polymer besteht, ausgeführt ist, implantierbare Carbonfasern zugesetzt sind.

3. Vorrichtung für die Koadaptation von Knochenteilen oder Knochenfragmenten nach Anspruch 1 oder Anspruch 2, **dadurch gekennzeichnet, dass** das erste Polymer einen härtenden Zusatz in einer Matrix aufweist, die von dem zweiten Polymer gebildet ist.

4. Vorrichtung für die Koadaptation von Knochenteilen oder Knochenfragmenten nach einem der Ansprüche 1 bis 3, **dadurch gekennzeichnet, dass** die mindestens eine Zone oder der Einsatz (2) aus Polyetheretherketon (PEEK) ausgeführt ist und das erste Polymer, aus dem der tragende Teil (4) außerhalb der mindestens eine Zone oder des Einsatzes ausgeführt ist, aus Polyetheretherketon (PEEK) ausgeführt ist, dem implantierbare Carbonfasern zugesetzt sind.

5. Vorrichtung für die Koadaptation von Knochenteilen oder Knochenfragmenten nach einem der Ansprüche 1 bis 4, **dadurch gekennzeichnet, dass** die Platte (1) auf der Unterseite im Bereich der mindestens eine Zone oder des Einsatzes (2) lokale Überdicken (5) aufweist.

6. Vorrichtung für die Koadaptation von Knochenteilen oder Knochenfragmenten nach einem der Ansprüche 1 bis 5, **dadurch gekennzeichnet, dass** mehrere Zonen oder Einsätze (2) vorgesehen sind.

7. Verfahren zur Herstellung einer Koaptationsvorrichtung nach einem der Ansprüche 1 bis 6, wobei in einer Form mindestens ein Einsatz (2) aus einem Polymermaterial gebildet wird, das biologisch kompatibel und formbar ist und ein anderes Polymermaterial, das biologisch kompatibel ist, eingespritzt wird, um einen tragenden Teil (4) einer Platte (1) der Koaptationsvorrichtung zu bilden, **dadurch gekennzeichnet, dass** das andere Polymermaterial eine Schmelztemperatur über der des biologisch kompatiblen formbaren Polymermaterials hat und um dieses biologisch kompatible formbare Polymermaterial nachdem und zur gleichen Zeit, in der der mindestens eine Einsatz (2) gebildet wird, unter Druck-, Dauer- und Temperaturbedingungen gespritzt wird, die derart sind, dass eine rheologische Kontinuität zwischen den zwei Polymermaterialien entsteht, die in einer teilweisen molekularen Verschmelzung zwischen den beiden Polymermaterialien resultiert, die durch eine nur oberflächliche Verschmelzung des mindestens einen Einsatzes beim Formen des tragenden Teils erhalten wird, so dass die Platte (1) einteilig erhalten wird.

8. Verfahren nach Anspruch 7, wobei sich die beiden Polymermaterialien nur dadurch unterscheiden, dass das andere Polymermaterial einen härtenden Zusatz enthält.

9. Verfahren nach Anspruch 7 oder Anspruch 8, wobei das Verfahren durch Aufformen durchgeführt wird und wobei:
- mindestens ein vorgeformtes Teil oder ein Einsatz (2), der aus deinem biologisch kompatiblen formbaren Polymer hergestellt ist, in einer Form platziert wird;
- um das vorgeformte Teil oder den Einsatz (2) ein Material in diese Form gespritzt wird, das aus demselben biologisch kompatiblen Polymer gebildet ist, dem aber Carbonfasern zugesetzt sind und das nach der Polymerisation/dem Formen eine höhere Steifigkeit als das vorgeformte Teil oder der Einsatz aufweist;
- man das Teil abkühlen lässt, bevor es herausgenommen wird.

10. Verfahren nach Anspruch 7 oder Anspruch 8, wobei das Verfahren durch Einspritzen von zwei Materialien durchgeführt wird und wobei:
- in eine Form, die mit zwei Einspritzpunkten für die Versorgung mit jedem der zwei Polymermaterialien ausgestattet ist, das biologisch kompatible und formbare Polymer für die Herstellung des mindestens einen Einsatzes (2) und ein Polymermaterial, das aus demselben biologisch kompatiblen Polymer gebildet ist, dem aber Carbonfaser zugesetzt sind, um den tragenden Teil (4) herzustellen, das nach der Polymerisation/dem Formen eine höhere Steifigkeit als der mindestens eine Einsatzes (2) aufweist, eingespritzt wird; und
- man die Vorrichtung abkühlen lässt, bevor sie herausgenommen wird

11. Verfahren nach einem der Ansprüche 9 bis 10, wobei das biologisch kompatible und formbare Polymermaterial, das den mindestens einen Einsatz (2) bildet, Polyetheretherketon (natürliches PEEK) ist und wobei das biologisch kompatible Material, das um den mindestens einen Einsatz (2) eingespritzt wird, um den tragenden Teil (4) zu bilden, PEEK ist, dem implantierbare Carbonfasern zugesetzt wurden.

12. Verfahren nach einem der Ansprüche 9 bis 11, wobei das Einspritzen in eine Form erfolgt, deren Temperatur zwischen 140 °C und 220 °C liegt und insbesondere bei einer Temperatur von 175 °C.

13. Verfahren nach einem der Ansprüche 9 bis 12, wobei das biologisch kompatible Polymer, dem Carbonfasern zugesetzt wurden, bei einer Temperatur zwischen 350 °C und 440 °C und insbesondere bei einer Temperatur von 395 °C eingespritzt wird.

14. Verfahren nach einem der Ansprüche 9 bis 13, wobei das biologisch kompatible Polymer, dem Carbonfasern zugesetzt wurden, mit einer Geschwindigkeit zwischen 50 g/sec und 750 g/sec und insbesondere mit einer Geschwindigkeit von 300 g/sec eingespritzt wird.

15. Verfahren nach einem der Ansprüche 9 bis 14, wobei das biologisch kompatible Polymer, dem Carbonfasern zugesetzt wurden, mit einem Druck zwischen 500 und 2000 bar und insbesondere mit einem Druck von 1000 bar eingespritzt wird.

16. Verfahren nach einem der Ansprüche 9 bis 15, wobei die erhaltene Vorrichtung eine Abkühlzeit benötigt, die zwischen 10 Sekunden und 30 Sekunden liegt und die Abkühlzeit der erhaltenen Vorrichtung insbesondere 20 Sekunden beträgt.

## Claims

1. A device for coaptation of bone parts or bone fragments, comprising a one-piece molded plate (1) comprising a support portion (4) produced from a first biocompatible polymer and comprising at least one zone or insert (2) provided with a through hole (3), said at least one zone or insert being produced from a second biocompatible polymer which is more malleable that the first polymer, said at least one zone or insert having mechanical properties enabling self-tapping of the inside surface of the holes, by means of screws usable for fastening said plate to bone tissues, the device being **characterized in that** the second polymer has a melting point that is lower than that of the first polymer, and **in that** the support portion (4) and the at least one zone or insert (2) present a rheological continuity therebetween resulting in a partial molecular melting together, which is achieved through a solely superficial melting of the at least one zone or insert upon molding of the support portion.

2. A device for coaptation of bone parts or bone fragments according to claim 1, **characterized in that** the first polymer from which is made the support portion (4), outside the at least one zone or insert (2) constituted by the second polymer, comprises a filler of implantable carbon fibers.

3. A device for coaptation of bone parts or bone fragments according to claim 1 or claim 2, **characterized in that** the first polymer comprises a hardening filler in a matrix formed by the second polymer.

4. A device for coaptation of bone parts or bone fragments according to one of claims 1 to 3, **characterized in that** the at least one zone or insert (2) is made from Polyetheretherketone (PEEK) and the first polymer from which is made the support portion (4), outside the at least one zone or insert, is made from Polyetheretherketone (PEEK) with a filler of implantable carbon fibers.

5. A device for coaptation of bone parts or bone fragments according to any one of claims 1 to 4, **characterized in that** the plate (1) has locally increased thicknesses (5) on the underside at the location of the at least one zone or insert (2).

6. A device for coaptation of bone parts or bone fragments according to any one of claims 1 to 5, **characterized in that** several zones or inserts (2) are provided.

7. A method of manufacturing a coaptation device according to any one of claims 1 to 6, comprising forming in a mold at least one insert (2) from a polymer material that is malleable and biocompatible and injecting another polymer material that is biocompatible to form a support portion (4) of a plate (1) of the coaptation device,
**characterized in that** said other polymer material has a melting point greater than that of the polymer material that is malleable and biocompatible and said other polymer material is injected around the polymer material that is malleable and biocompatible after or at the same time as the at least one insert (2) is formed, under conditions of pressure, time and temperature such that rheological continuity occurs between the two polymer materials resulting in a partial melting together of the two polymer materials, which is achieved through a solely superficial melting of the at least one insert upon molding of the support portion, so as to obtain the plate (1) as one-piece.

8. A method according to claim 7, wherein the two polymer materials differ only in the fact that said other polymer material contains a hardening filler.

9. A method according to claim 7 or claim 8, wherein the method is implemented by overmolding, and wherein the method comprises:
- placing in a mold, at least one preformed part or insert (2) made from the polymer material that is malleable and biocompatible;
- injecting into that mold, around said preformed part or insert (2), a material formed from the same polymer material but with a Carbon fiber filler, and having, after polymerization/molding, a higher rigidity than that of said preformed part or insert; and
- leaving the device to cool before ejecting it.

10. A method according to claim 7 or claim 8, wherein the method is implemented by bi-material injection, and wherein the method comprises:
- injecting into a mold, provided with two injection points to supply each of the two polymer materials, the polymer material that is malleable and biocompatible to manufacture said at least one insert (2) and a material formed from the same polymer material but with a carbon fiber filler to manufacture the support portion (4), having after polymerization/molding a higher rigidity than that of the at least one insert (2); and
- leaving the device to cool before ejecting it.

11. A method according to any one of claims 9 to 10, wherein the malleable and biocompatible polymer material that constitutes the at least one insert (2) is Polyetheretherketone (natural PEEK), and wherein the biocompatible material injected around the at least one insert (2) to constitute the support portion (4) is PEEK with a filler of implantable carbon fibers.

12. A method according to any one of claims 9 to 11, wherein the injection is made in a mold of which the temperature is comprised between 140°C and 220°C, and more particularly at a temperature of 175°C.

13. A method according to any one of claims 9 to 12, wherein the biocompatible polymer material with the carbon fiber filler is injected at a temperature comprised between 350°C and 440°C, and more particularly at a temperature of 395°C.

14. A method according to any one of claims 9 to 13, wherein the biocompatible polymer material with the carbon fiber filler is injected at a speed comprised between 50 g/sec and 750 g/sec, and more particularly at a speed of 300 g/sec.

15. A method according to any one of claims 9 to 14, wherein the biocompatible polymer material with the carbon fiber filler is injected at a pressure comprised between 500 and 2000 Bars, and more particularly at a pressure of 1000 Bars.

16. A method according to any one of claims 9 to 15, wherein the device obtained requires a cooling time comprised between 10 seconds and 30 seconds, and more particularly the cooling time of the device obtained is 20 seconds.
